# EUROPEAN PATENT APPLICATION

(11) **EP 2 649 940 A1**
(43) Date of publication of application: **16.10.2013**
(21) Application number: 13176198.3
(22) Date of filing: 25.02.2009
(51) Int. Cl.: A61B 6/00, G03B 42/04

(54) **Radiography system**

(30) Priority: 18.09.2008 JP 2008239118
(62) Divisional of application: 09814339.9
(71) Applicant: Konica Minolta Medical & Graphic, Inc., Tokyo 191-8511 (JP)
(72) Inventor: Misawa, Takao, Tokyo, 191-8511 (JP); Arita, Kazuhiro, Tokyo, 191-8511 (JP)
(74) Representative: Burton, Nick

(57) **Abstract**

Provided is a radiography system, which can use a CR cassette and an FPD mixedly together and which can limit the influence range accompanying the addition or updating, thereby expanding the system function easily. On the basis of an input of photographing instructions that a radiation image detecting device (6) is used by an operator to an operation unit (78), a control terminal (7) notifies a managing means (4) of photographing instructions to use the radiation image detecting device (6) by the operator. On the basis of that notification, the managing means (4) controls an irradiation device (3) and the radiation image detecting device (6) thereby executing the photography.

## Description

### FIELD OF THE INVENTION

The present invention relates to radiography systems, and to radiography systems that utilize portable type radiation image detecting devices.

### DESCRIPTION OF RELATED ART

In medical diagnosis locations, CR (Computed Radiography) systems have been realized that use a reading device that reads radiation image data by scanning using an excitation light, a fluorescent material plate incorporated inside a CR cassette, and a control device (console) that acquires the radiation image data read by that reading device (see Patent Reference 1).

Furthermore, instead of the above mentioned CR cassette, Flat Panel Detectors (FPD) that have built into them radiation detection elements arranged in a two dimensional array on a substrate and capable of outputting electrical signals corresponding to the amount of radiation impinging on those radiation detection elements have been proposed as radiation image detecting devices. If such an FPD is used, since it is not necessary to use a reading device that reads out the radiation image by emitting an excitation light, and since it is possible to acquire the radiation image data directly, it becomes possible to make the entire system compact compared to when using a CR cassette, and also, even the photographing operation becomes smooth.

Because of these merits, there is a demand for replacing existing CR systems constructed using CR cassettes with FPDs. In particular, small size and lightweight have become possible due to advances in semiconductor technology, and such replacement is possible using a cassette type FPD cassette that is of the portable type with a size equivalent to that of CR cassettes and provided with a wireless communication section.

In this case, since it is possible to utilize the radiation emission section that emits radiation and that was being used with the CR cassettes and the photographing table in which the CR cassette is mounted (the so called bucky apparatus) as they are, there is the merit that it is possible to suppress the cost of introduction.

However, in an existing system, it is difficult to replace with a system having all FPDs at the same time from the point of view of cost. Because of this, when replacing, a mixed type is desirable of introducing new FPDs while using the existing CR system. In a mixed type, in order to eliminate the confusion caused to operators such as radiology technicians, it is desirable that a new control terminal is not installed separately but the control terminal being used in the conventional CR system is used as it is.

In Patent Reference 2 a radiation imaging system in which even when radiation imaging devices of different types and different detection means are mixed, the control is carried out by a single control device has been disclosed.

Patent Reference 1: Unexamined Japanese Patent Application Publication No. 2002-158820.

Patent Reference 2: Unexamined Japanese Patent Application Publication No. 2001-149358.

### SUMMARY OF THE INVENTION

### PROBLEMS TO BE SOLVED BY THE INVENTION

When carrying out radiography using an FPD, it is necessary not only to change the amount of radiation emitted by the radiation emitting device, but also to coordinate the radiation emission timing with a sequence of operation timings for acquiring radiation image data of the FPD, the sequence of operation includes, for example, the resetting operation, etc. In order to achieve such timing coordination, it is necessary to select in advance in the control terminal the FPD to be used for radiography, and to establish a communication link among the FPD, the control device, and the radiation emitting device.

Because of this, in order to introduce a system using FPDs, it is not sufficient to introduce only the FPD alone, but it is necessary to make even the control device and the radiation emitting device compatible with the introduced FPD, and to change the specifications of the hardware configuration and software configuration, etc.

In order to achieve functional extension of a radiography system, when the version of specifications of the apparatuses of a part of the system is upgraded or when a new configuration is introduced, in order that its effect is present on other apparatuses as well, it may become necessary to change the specifications of the entire system.

In this manner, the debugging evaluation associated with the renewing of a part of the equipment, installation of new software, or version upgrading of existing software in order to achieve functional expansion of the radiography system is complex and also large in size, and becomes one that requires considerable labor and time. In addition, in the case of large scale medical systems, there are cases in which system updating becomes necessary of a large scale system including an RIS (Radiology Information System) or an HIS (Hospital Information System).

Considering the above problems, the present invention has the purpose of, even when an FPD is added to a radiography system using CR cassettes, making it possible for both of them to be present in a mixed manner, and also, limiting the range of effect due to addition or updating, making it possible to reduce the scope of system debugging at the time of functional expansion of the system, and making it possible to carry them out easily.

### MEANS FOR SOLVING THE PROBLEMS

1. A radiographic imaging system comprising: a portable type radiation image detecting device that carries out radiography based on a radiation from a radiation emitting device provided in a radiography room and obtains radiation image data; a control terminal which is provided with an operation section and a display section, acquires radiography order information, and establishes correspondence between the acquired radiography order information and radiation image data generated by said radiation image detecting device; and a managing means which controls at least one radiation emitting device of the radiation emitting device, wherein each of said radiation image detecting device, said control terminal and said managing means is possible to mutually communicate via communication lines, wherein said control terminal sends notification of a radiography instruction to said managing means, based on an input of a radiography instruction by an operator to the operating section, the radiography instruction is an instruction to the effect that said radiation image detecting device is to be used; and wherein said managing means carries out radiography by controlling the radiation emitting device or controlling the radiation emitting device and said radiation image detecting device based on the notification.
2. The radiation imaging system according to 1 above, wherein said managing means controls a timing of emission of radiation of said radiation emitting device and a timing of obtaining radiation image data of said radiation image detecting device.
3. The radiation imaging system according to 1 or 2 above, wherein said managing means is capable of controlling a plurality of the radiation emitting devices, wherein said control terminal sends notification of the radiography instruction and a selection instruction of the radiation emitting device to be used for radiography to said managing means, based on an input of the radiography instruction and an input of the selection instruction of the radiation emitting device to be used by an operator to the operating section; and wherein said managing means carries out radiography by controlling the selected radiation emitting device and said radiation image detecting device based on the notification.
4. The radiation imaging system according to any one of 1 to 3 above, wherein the radiographic imaging system comprises a plurality of radiation image detecting devices to which identification IDs are assigned, wherein said control terminal sends notification of the radiography instruction and a selection instruction of a radiation image detecting device to be used for radiography to said managing means, based on an input of the radiography instruction and an input of the selection instruction of the radiation image detecting device to be used by an operator to the operating section; and wherein said managing means carries out radiography by controlling the radiation emitting device and the selected radiation image detecting device based on the notification.
5. The radiation imaging system according to any one of 1 to 4 above, wherein said radiation image detecting device is provided with a wireless communication section that can carry out wireless communication; wherein said managing means, executes radiography by controlling said radiation image detecting device through wireless communication; and wherein said radiation image detecting device carries out transmission of the generated radiation image data to said control terminal by wireless communication.
6. The radiation imaging system according to any one of 1 to 5 above, wherein the radiographic imaging system comprises a plurality of said control terminals and at least one of the plurality of control terminals and said managing means are connected by a wireless communication circuit.
7. The radiation imaging system according to 1 above, wherein said radiation image detecting device comprises: a plurality of detection elements that convert an energy corresponding to an amount of radiation impinging on them into an amount of electrical charge; a bias line that applies a reverse bias voltage to each of said detection elements; a power supply that applies a reverse bias voltage to said detection elements via said bias line; a current detection means that detects a current flowing through said bias line; and a control means that detects a start and an end of emission of said radiation based on an increase or a decrease in the current flowing through said bias line detected by said current detection means, wherein said managing means, based on said notification, carries out notification of a reset instruction to said radiation image detecting device.
8. The radiation imaging system according to 1 above, wherein said radiation image detecting device comprises: a plurality of detection elements that convert an energy corresponding to an amount of radiation impinging on them into an amount of electrical charge; a bias line that applies a reverse bias voltage to each of said detection elements; a power supply that applies a reverse bias voltage to said detection elements via said bias line; a current detection means that detects a current flowing through said bias line; and a control means that detects a start and an end of emission of said radiation based on an increase or a decrease in the current flowing through said bias line detected by said current detection means, wherein said control terminal, based on said notification, carries out notification of a reset instruction to said radiation image detecting device.

### ADVANTAGES OF THE INVENTION

According to the present invention, even when an FPD is added to a radiography system using CR cassettes, by making the configuration such that a control box is added corresponding to the imaging device, it becomes possible that both of them are present in a mixed manner, and also, the range of effects due to the addition of updating is made limited, and it is possible to reduce the scope of system debugging at the time of functional expansion of the system, and to carry it out easily.

### BRIEF DESCRIPTION OF THE DRAWINGS

Fig. 1 is a diagram showing an outline configuration of a radiography system in a first embodiment.
Fig. 2 is a diagram showing an outline configuration of a radiography system in a second embodiment.
Fig. 3 is a diagram showing an outline configuration of a radiography system in a third embodiment.
Fig. 4 is a diagram showing an outline configuration of a radiography system in a fourth embodiment.
Fig. 5 is a block diagram showing the important constitution of a console 7.
Fig. 6 is a block diagram showing the important constitution of an FPD 6.
Fig. 7 is a perspective view diagram of an FPD 6.
Fig. 8 is a schematic diagram showing an imaging panel 62 and the circuit configuration around it.
Fig. 9 is a diagram for explaining the control flow executed by the radiography system.
Fig.10 is an example of the settings screen displayed in the display section 77.
Fig. 11 is a schematic diagram showing an imaging panel 62 and its peripheral circuit configuration according to a fifth and a sixth embodiment.
Fig. 12 is a cross-sectional view diagram of the imaging panel 62 shown in Fig. 11.
Fig. 13 is a diagram showing an example of the change with time in the voltage value that is converted and output from the current value in the current detection means 634.
Fig. 14 is a diagram for explaining the control flow executed by the radiography system according to the fifth embodiment.
Fig. 15 is a diagram for explaining the control flow executed by the radiography system according to the sixth embodiment.

### LEGEND

100 Radiography room
11 Radiography table
2 Radiation image reading device
3 Radiation emitting device
35 Operation button
4 Control box
5 Access point
6 FPD
7 Console
9 CR Cassette

### DETAILED DESCRIPTION OF THE PREFERRED EMBODIMENTS

While the present invention is described below based on some embodiments, the present invention is not limited to these embodiments.

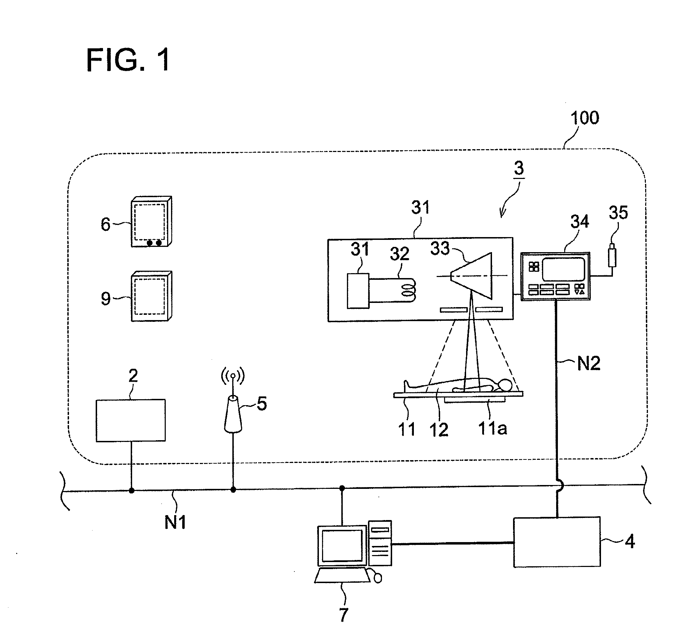

Fig. 1 is a diagram showing an outline configuration of a radiography system in a first embodiment As is shown in this figure, a radiography system is configured to have a radiation image reading device 2, a radiation emitting device 3, a control box 4, an access point 5, and a console 7, etc. The radiation image reading device 2, the radiation emitting device 3, the access point 5, and die radiographing table 11 are provided inside the radiography room 100. Further, the control box 4 in the present embodiment functions as a "managing means", and the console 7 functions as a "control terminal".

The number 6 refers to a radiation image detecting device (hereinafter referred to merely as an FPD 6) which has incorporated inside it radiation detecting elements arranged in a two dimensional array on a substrate, and it is possible to obtain the radiation image data directly. The number 9 refers to a CR cassette as a device of a type different from the FPD 6. After radiation is emitted on to the CR cassette 9, if it is loaded into a radiation image reading device 2, the fluorescent plate built in the CR cassette 9 is scanned by an excitation beam of light and the radiation image is read out.

The access point 5 for carrying out wireless communication using a wireless LAN (Local Area Network) with devices such as the FPD 6, etc., and the console 7 (control terminal) that carries out computation processing or display processing of the radiation image data obtained from the radiation image detecting device 6 are directly connected using a network N1 forming this configuration. Although the network N1 can be a communication circuit dedicated to that system, because of reasons such as the degree of freedom of the system configuration becomes low, etc., it is desirable to use existing lines such as Ethernet (Registered trademark), etc.

The console 7, the radiation emitting device 3, and the control box 4 are configured by connection using a network N2. In the present embodiment, the network N2 are communication lines dedicated to that system, and can carry out communication directly or indirectly with a device of the network N1 either via the console 7 or by the console 7 carrying out protocol conversion. By having a configuration such as that in the present embodiment, it is possible to make the system one in which a portable type FPD can be mixed by adding the communication lines of the network N2 to the existing system using CR cassettes.

Further, in this system although showing in the figure is irradiated, connection is made with an HIS that centrally manages the patient diagnostic information or accounting information, or with an RIS that manages radiological diagnostic infomlation via the network N1.

The access point 5, when wireless communication is made an FPD 6 provided inside the radiography room in which is provided the radiation emitting device 3 and the console 7, has the function of relaying the communication between them. Further, although the example described here is one in which the wireless communication is being carried out using a wireless LAN (for example, a communication method compatible with IEEE 802.11a/b/g), it is not necessarily restricted to this, and it is also possible to carry out wireless communication, apart from using radio waves (special waves), by carrying out optical wireless communication using infrared or visible light rays (laser, etc.), etc. (for example, IrDA), or by carrying out sonic communication using sonic waves or ultrasonic waves.

### [Radiation Emitting Device 3]

The radiation emitting device 3 emits radiation towards a patient 12 lying down on a radiography table 11, and on the under side of the radiography table 11 is provided a loading slot 11a for inserting an FPD 6 or a CR cassette 9.

The radiation emitting device 3 is constituted to have a high voltage power supply 31 that applies a high voltage between a filament 32 and another filament 32, a rotating anode 33, an operation section 34, and operation buttons 35. The rotating anode 33 is also called a rotor or a target, is made of a heavy metal, and is rotated at a prescribed rotational speed by a driving motor not shown in the figure. By applying a high voltage, for example, 20 kV to 150 kV, from the high voltage power supply 31 to the filaments 32, X-rays are generated because an electron beam is accelerated towards and made to impinge on the rotating anode 33.

Settings of the range of X-ray irradiation and the amount of X-ray irradiation are made in the operation section 34. Further, it is also possible to configure so that these settings are made automatically based on the body part information, etc. included in the radiography order information. The radiography using a CR cassette 9, etc. is carried out in the standard mode in which synchronization between the radiation emitting device 3 and the imaging medium is not required. In the standard mode, by a radiography technician or some other person pressing the operation button 35, the emission of X-ray radiation is started towards the person under examination 12 lying on the radiography table 11. At this time, the rotation of the anode 33 is started due to a signal generated due to the pressing of the operation button 35, and after it reaches the prescribed rotational speed and the rotation becomes stable, a high voltage is applied to the filaments 32 thereby starting the emission of X-ray radiation. Further, it is also possible to configure so that the operation button 35 can be pressed in two stages, so that the rotation of the rotating anode 33 is started when the first half pressing is made, and when the succeeding second half pressing is made, the high voltage is applied to the filaments 32 thereby starting the emission of X-ray radiation.

When using the FPD 6, the standard mode is not used since synchronization is necessary, but it is necessary to execute using the FPD mode. When using the FPD mode, it is necessary to control the timing of emitting the X-ray radiation from the radiation emitting device 3 during radiography and the timing of acquiring the radiation image data of the FPD 6. In other words, before the radiation is emitted, it is necessary that the resetting of the FPD 6 is completed, the FPD 6 is in a state in which accumulation of imaging data is possible, and the reading of the radiation image is started at the end of emitting the radiation. In the following, this control is simply referred to as "synchronization" and the details are described later.

Further, it is desirable that the irradiation conditions, etc. of the radiation emitting device at the time of using a CR cassette 9 are made suitable for the sensitivity characteristics, etc. of the CR cassette 9. In the present embodiment, during the radiography using a CR cassette 9, it is also possible to identify the identification code ID, etc. of the CR cassette 9 by giving input instruction to the operator of the console 7, and the data of irradiation conditions, etc., with the CR cassette 9 is transmitted to the operation section 34 via the network N2.

Further, although the selection of using either a CR cassette 9 or an FPD 6 for carrying out radiography is made in the console 7, in the former case, it is also possible to configure so that the network N2 that is not used is switched OFF, and the network N1 is switched OFF in the latter case.

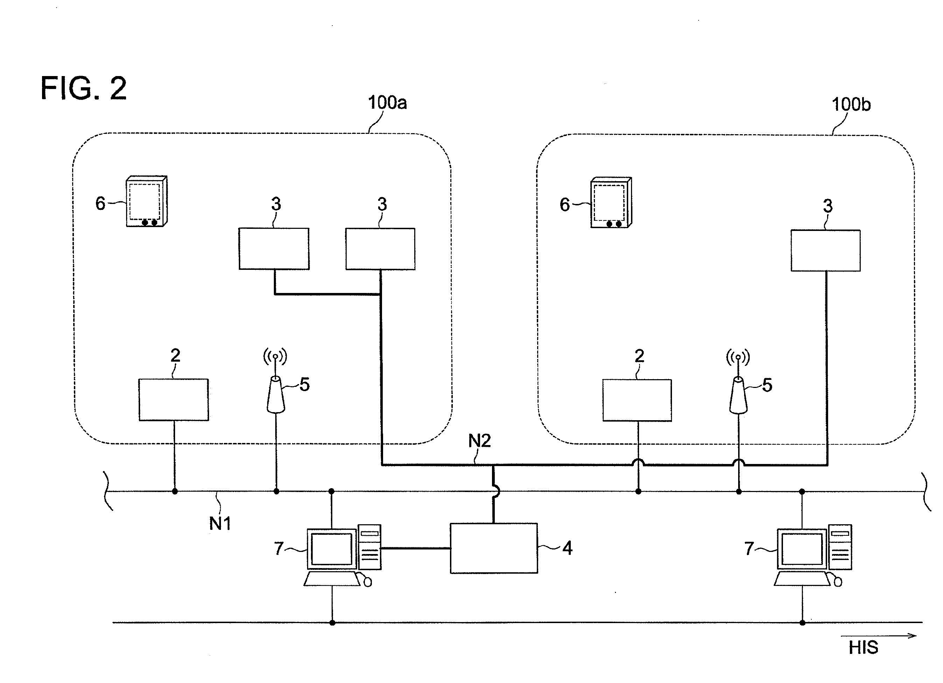

The radiography systems according to other embodiments are explained based on Fig. 2 to Fig. 4. Fig. 2 to Fig. 4 are diagrams showing the outline configurations of radiography systems according to the second to the fourth embodiments. In these embodiments, explanations of the same configuration parts as those in the embodiment shown in Fig. 1 are substituted by assigning the same symbols. Further, regarding these figures, the descriptions of the configurations of each of the radiation emitting devices 3 and of the respectively corresponding radiography tables 11 are omitted.

Although in the first embodiment, an example was shown in which each control box 4 was connected respectively to one radiation emitting device 3 and one console 7, in the embodiment shown in Fig. 2, one control box 4 has been connected to a plurality of radiation emitting devices 3, and control of this plurality of radiation emitting devices 3 is possible. Further the control box 4 is connected to a plurality of consoles 7. By mediating through a control box 4, it is possible to operate the radiation emitting device 3 or the FPD 6 that are inside any one of the radiography rooms 100a and 100b.

Further, although not shown in the figure here, in the radiography system of Fig. 2, connection has been made with an HIS that centrally manages the patient diagnostic information or accounting information, or with an RIS that manages radiological diagnostic information via the network N1.

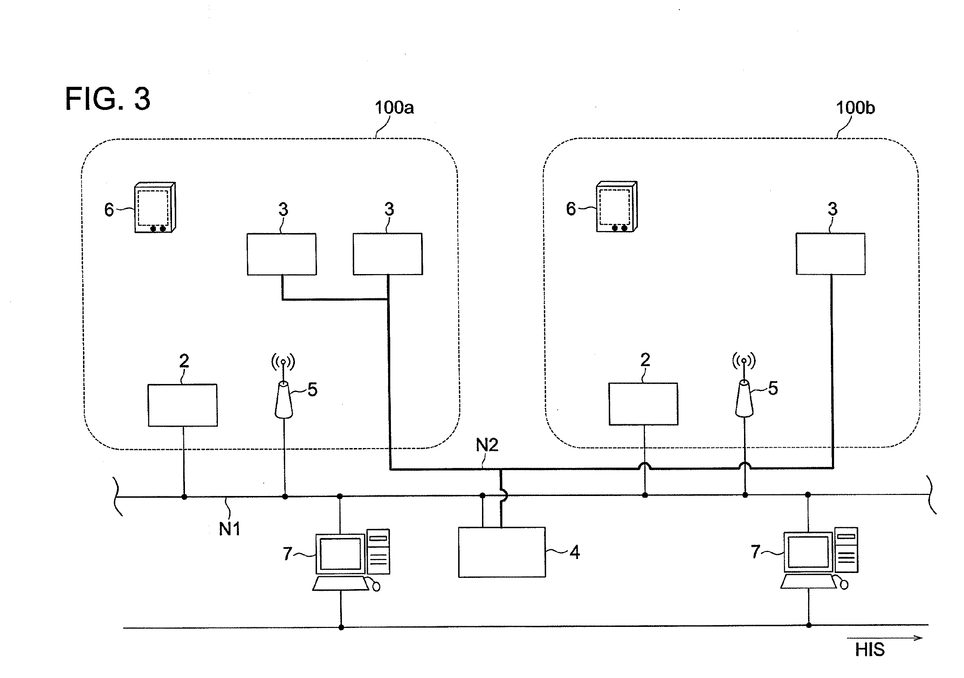

In the third embodiment shown in Fig. 3, the control box 4 is connected directly with the network N1, and can carry out direct communication with a plurality of consoles 7.

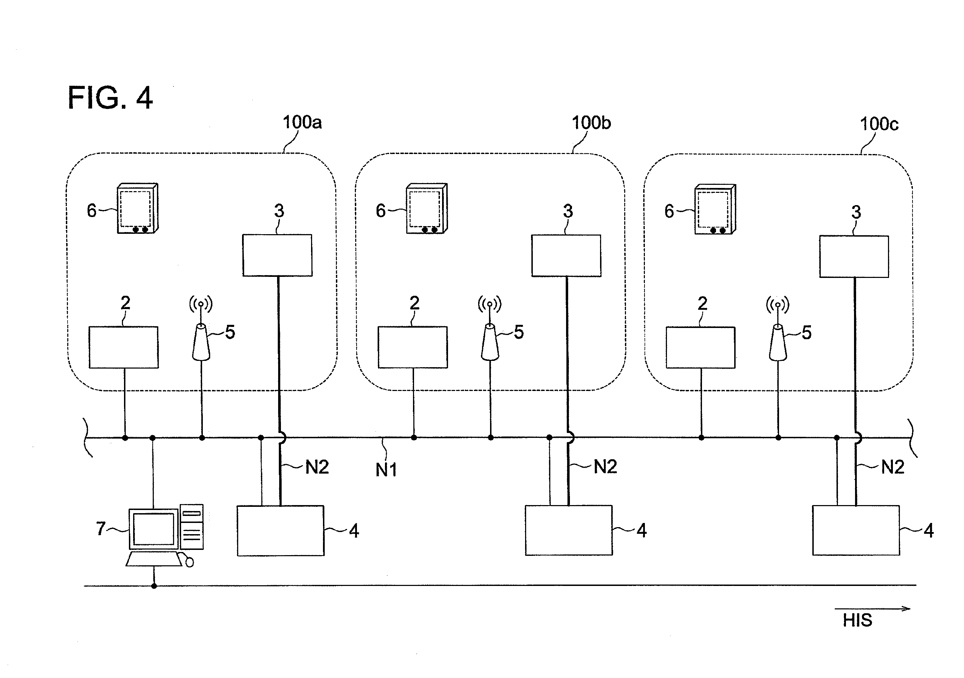

In the fourth embodiment shown in Fig. 4, a control box 4 is placed inside each radiography room 100, and also the console 7 is shared commonly among the radiography rooms 100a, 100b, and 100c.

### [Console 7]

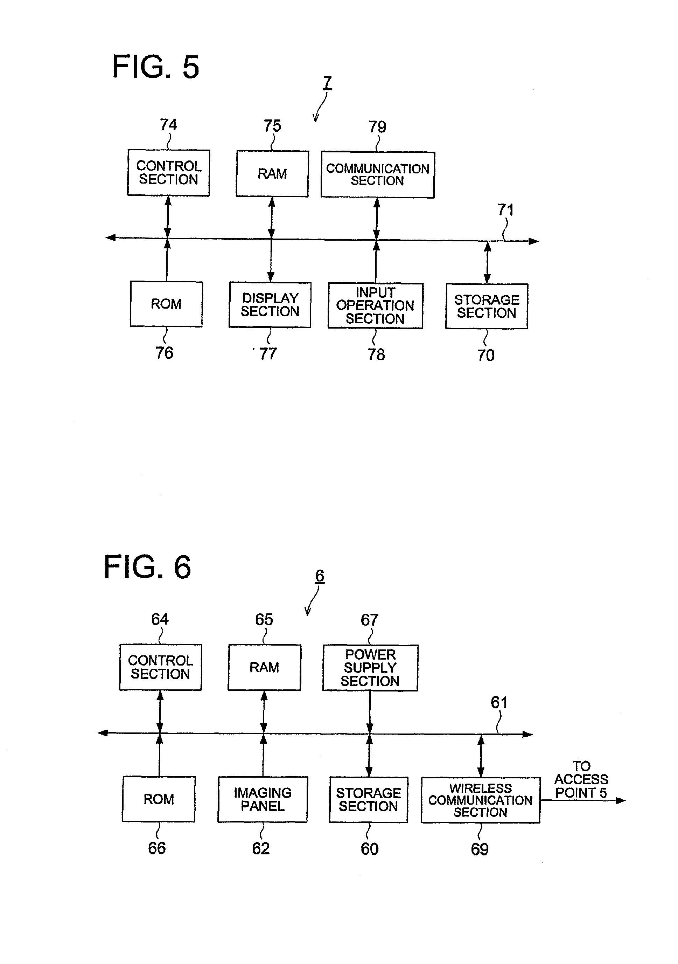

Fig. 5 is a block diagram showing the important configuration parts of a console 7. The console 7, as is shown in Fig. 5, is configured to be provided with a control section 74, a RAM (Random Access Memory) 75, a ROM (Read Only Memory) 76, a display section 77, an input operation section 78, a communication section 79, and a storage section 70, etc., and each of these parts are connected by a bus 71.

The display section 77, for example, is configured to be provided with a CRT (Cathode Ray Tube) or an LCD (Liquid Crystal Display), etc., and displays various screens such as the radiography order, the patient list, and various types of messages or images, etc., according to the instruction of the display signal transmitted from the control section 74.

The input operation section 78, for example, is configured to have a keyboard, a mouse, etc., and outputs as the input signal to the control section 74 the depression signal of key pressed in the keyboard or the mouse operation signal. Further, the input operation section 78 can also be configured as a so called touch panel that outputs as the input signal to the control section 74 the position information input by touching with a finger or a dedicated stylus pen the transparent sheet panel covering the surface of the display screen of said display section 77.

In particular, in the radiography system according to the present embodiment, the input operation section 78 displays the cassette ID of the FPD 6 at the time of inputting the input of the radiography order information such as the patient information corresponding to the radiography which is to be made, or the radiography information, etc., and at the time of establishing correspondence between that radiography order information and the radiographed radiation image data, and in addition it can also be made to display a correspondence establishment confirmation screen at that time. Here, the cassette ID (also called the identification code ID) is some unique identification information assigned to each FPD 6 for identifying the FPD 6.

Further, in the case of a portable FPD 6, there are situations when it is not possible to determine in which radiography room that particular FPD 6 is present. Considering such a situation, it is also possible to configure so that "position detection" is carried out, and its result is displayed in the display section 77.

As a method of "position detection", a communication request directed at that FPD 6 is transmitted from the console 7 to all the FPDs 6. In concrete terms, as "simultaneous paging (called polling)", a communication request is made via all the access points 5 in all the radiography rooms, and the position detection of the called FPD 6 is made from the path information such as the IP address, etc. of the route access point 5 added to the communication data in that response.

### [FPD 6]

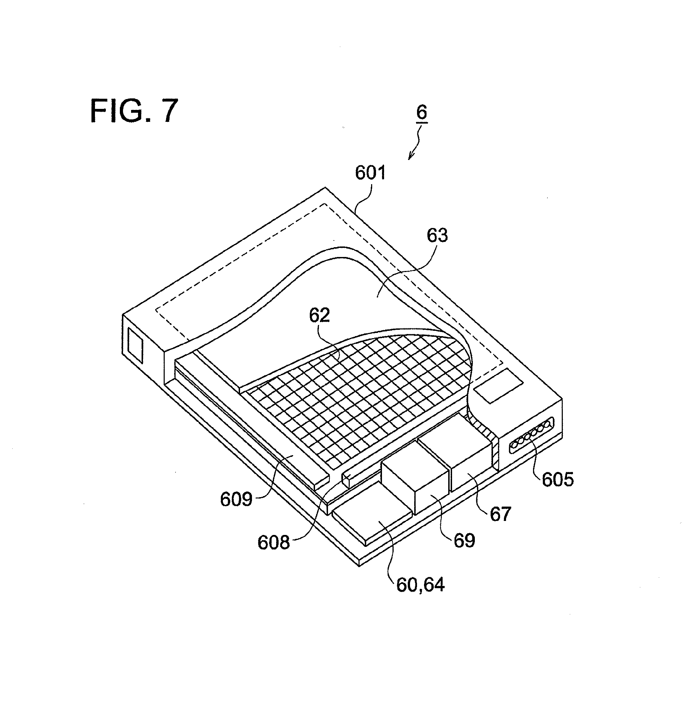

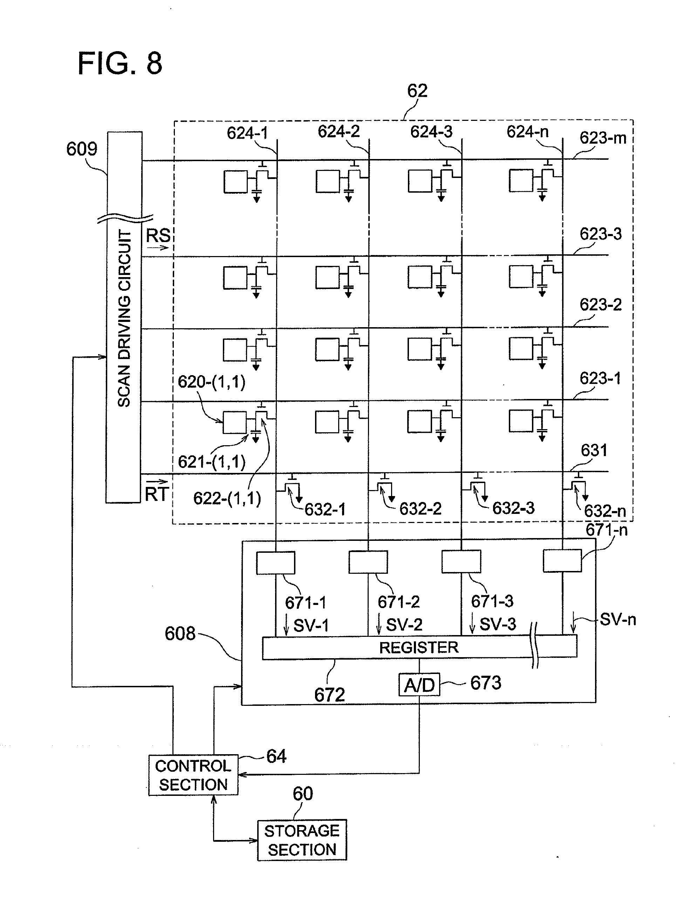

An FPD 6 as a radiation image detecting device is explained based on Fig. 6 to Fig. 8. Fig. 6 is a block diagram showing the main constituent parts of an FPD 6 and Fig. 7 is a perspective view diagram of the FPD 6. Fig. 8 is a schematic diagram showing the imaging panel 62 and its peripheral circuit configuration.

As is shown in Fig. 6, the control system of the FPD 6 is provided with a control section 64, a RAM 65, a ROM 66, an imaging panel 62, a storage section 60, a power supply section 67, and a wireless communication section 69, etc., each of which are connected by a bus 61.

The control section 64 functions as a managing means, and is configured, for example, to have a CPU, etc., which reads out the control program stored in the ROM 66 and expands it in the work area formed inside the RAM 65, and controls the different parts of the FPD 6 in accordance with that control program. The ROM 66 is constituted from a nonvolatile semiconductor memory, etc., and stores the control program executed by the control section 64, various types of programs, and the cassette ID of the FPD 6, etc. The RAM 65, during the different types of processing controlled and executed by the control section 64 constitute a work area that temporarily stores various programs read out from the ROM 66 and that can be executed by the control section 64, input or output data, and parameters, etc.

The storage section 60 is constituted from, for example, a non volatile memory such as a flash memory, or a RAM, etc., and can store the radiation image data corresponding to several times of radiography and obtained by reading out the electrical signals stored in the imaging panel 62.

The wireless communication section 69 is one that carries out wireless communication of various types of information via a wireless LAN conforming to IEEE standard 802.11 and via the access points 5 with the consoles 7.

The power supply section 67 acts as a battery supplying electric power to the plurality of drive sections (the control section 64, the imaging panel 62, the storage section 60, etc.) constituting the FPD 6. This power supply section 67 is configured, for example, as a spare battery and a rechargeable battery that can be charged freely, and the rechargeable battery can be charged by connecting the connector 605 to a cradle not shown in the figure.

As is shown in Fig. 7, the FPD 6 is provided with a body 601 for protecting its inside, and is constituted as a cassette that can be transported. Inside the body 601 is formed a layer of the imaging panel 62 that converts the radiation emitted on to it into an electrical signal. On the surface of this imaging panel 62 that is irradiated with radiation is provided a light emitting layer 63 that emits light according to the strength of the incident radiation.

The light emitting layer 63 is one that is generally called the scintillating layer, has, for example, a phosphor as the main constituent, and outputs electromagnetic waves in the wavelength range of 300 nm to 800 nm based on the incident radiation, that is, from ultraviolet to infrared light electromagnetic radiation (light) centering on visible light.

On the surface of this light emitting layer opposite to the surface which is irradiated with radiation, is formed an imaging panel 62 in which are arranged in the form of a matrix a plurality of optoelectronic conversion sections that convert the electromagnetic waves (light) output from the light emitting layer into electrical energy and accumulate it, and output an image signal based on that accumulated electrical energy. Further, the signal output from one optoelectronic conversion section becomes a signal corresponding to one pixel, which is the smallest unit constituting the radiation image data. In addition, the imaging panel 62 has a scan driving circuit 609 that reads out the accumulated electrical energy, and a signal selection circuit 608 that outputs the accumulated electrical energy and the image signal.

Here, the circuit configuration of the imaging panel 62 is explained based on Fig. 8. As is shown in this figure, the imaging panel 62 has a plurality of light receiving elements 620 (also called detection elements) that are arranged in two dimensions and that convert light into electrical signals, and one light receiving element corresponds to one pixel of the radiation image. These pixels have a density of, for example, 200 to 400 dpi (dots per inch), and are placed over the size of the imaging area of the body under examination.

Further, between the light receiving elements 620 are placed scanning lines (horizontal lines) 623 and signal lines (vertical lines) 624, and in this figure, they are arranged in the form of a grid so that they intersect each other at right angles. Here, if one segment enclosed between the scanning lines 623 and the signal lines 624 is taken as one pixel, the number of pixels of the imaging panel 62 is constituted from, for example, m x n pixels when m of them are arranged in one direction and n of them are arranged in the other direction. Further, in the imaging panel 62, the photodiodes 621-(1, ) to 621-(m, n) and the transistors 622-(1, 1) to 622-(m, n), which are switching elements, have been arranged corresponding to m x n number of pixels, and the scanning lines 623-1 to 623-m and the signal lines 624-1 to 624-n will be arranged between the pixels so that they intersect at right angles to each other.

For example, in the first light receiving element, to the photodiode 621-(1, 1) is connected the transistor 622-(1, 1) that is a switching element configured with a silicon multilayer structure or an organic semiconductor. A field effect transistor, for example, is used for the transistor 622-(1, 1). Not only the drain electrode or the source electrode of the transistor 622-(1, 1) is connected to the light receiving element 620-(1, 1) but the gate electrode of the transistor 622-(1, 1) is also connected to the scanning line 623-1. When the drain electrode is connected to the light receiving element 620-(1, 1), the source electrode is connected to the signal line 624-1, and when the source electrode is connected to the light receiving element 620-(1, 1), the drain electrode is connected to the signal line 624-1. Further, even the light receiving elements 620, the photodiodes 621, and the transistors 622 of other pixels are connected similarly to the scanning line 623 and the signal lines 624.

In addition, imaging panels 62 are also provided with initializing transistors 632-1 to 632-n whose drain electrodes are connected to the signal lines 624-1 to 624-n as shown in Fig. 8, and the source electrodes of these initializing transistors 632-1 to 632-n are connected to the ground, and the gate electrodes are connected to the reset line 631.

In the imaging panel 62, the radiation image is converted into digital image signals through these circuits. In other words, the control section 64 scans the image by supplying the read signal RS via the scan driving circuit 609 to each of the scanning lines 623-1 to 623-m, reads the digital image signal for each scanning line, and converts the radiation image into digital image signals. This is explained in detail below.

The scanning lines 623-1 to 623-m and the reset line 631 of the imaging panel 62 are connected to the scan driving circuits 609 as shown in Fig. 8. Among the scanning lines 623-1 to 623-m from the scan driving circuits 609, when the read signal RS is supplied to any required scanning line 623-p (where p is any value from 1 to m), the transistors 622-(p, 1) to 622-(p-n) connected to this scanning line 623-p go into the ON state, and the charges stored in the photodiodes 621-(p, 1) to 621-(p, n) are output to the signal lines 624-1 to 624-n.

The signal lines 624-1 to 624-n are connected to the signal converters 671-1 to 671-n of the signal selection circuits 608, the voltage signals SV-1 to SV-n corresponding to the amount of charge output on the signal lines 624-1 to 624-n are output by the signal converters 671-1 to 671-n, and the voltage signals SV-1 to SV-n output by the signal converters 671-1 to 671-n are supplied to the register 672.

The register 672 successively selects the voltage signal supplied by the signal converter 671, and the selected voltage signal is converted into one 12-bit to 14-bit digital image signal by the analog to digital (A/D) converter 273, this digital image signal is supplied to the control section, and the radiation image is converted into digital image signal in units of a pixel.

Further, when carrying out initialization of the imaging panel 62, at the beginning, after a reset signal RT is supplied from the scan driving circuit 609 to the reset line 631 and the initialization transistors 632-1 to 632-n are put in the ON state, the read signal RS is supplied to the scanning lines 623-1 to 623-m putting the transistors 622-(1, 1) to 622-(m, n) in the ON state. Next, by discharging the charges accumulated in the photodiodes 621(1, 1) to 621(m, n) through the initializing transistors 632-1 to 632-n, the imaging panel 62 is initialized.

### [Synchronization between the Radiation Emitting Device 3 and the FPD 6 during Imaging]

In the FPD 6, even when no radiation is being emitted, the photodiodes 621 accumulate very small quantities of electric charge, that is, a so called dark current is generated. The electric charge accumulated in the photodiodes 621 due to this dark current phenomenon becomes noise while obtaining the radiation image data and has a bad effect on the image data. In order to make the effect of this dark current as low as possible, it is effective to purge the accumulated electric charge just before carrying out imaging. In order to achieve both suppressing the X-ray radiation dose of the patient 12 to as small a value as possible and suppressing the effect of dark current, it is necessary to establish synchronization between the radiation emitting device 3 and the FPD 6 during imaging. This synchronization is that of carrying out (1) resetting the charges on the photodiodes 621 of the FPD 6 just before emitting radiation from the radiation emitting device 3 (charge reset state), (2) putting the photodiodes 621 in the accumulation enabled state matching with the start of radiation emission, and (3) starting the reading of the charges accumulated in the photodiodes 621 (charge reading state) matching with the stopping of emission of radiation (end of irradiation).

Here, explanations are given about the different states of the "charge reset state", the "charge reading state", and the "accumulation enabled state". The "charge reset state" is the state in which initialization described above is executed successively at prescribed intervals, the "charge reading state" is the state in which the electric charge accumulated in each of the photodiodes 621 is successively output to the signal line and converted into a digital image signal via a register 672, and the "accumulation enabled state" is the state in which the above initialization or reading are not done but the charge converted due to emission of radiation is continued to be accumulated in each of the photodiodes 621, that is, the so called state in which exposure is being made. Further, the control in these states is executed by the control section 64 under the management of the control box 4.

### [Control Flow]

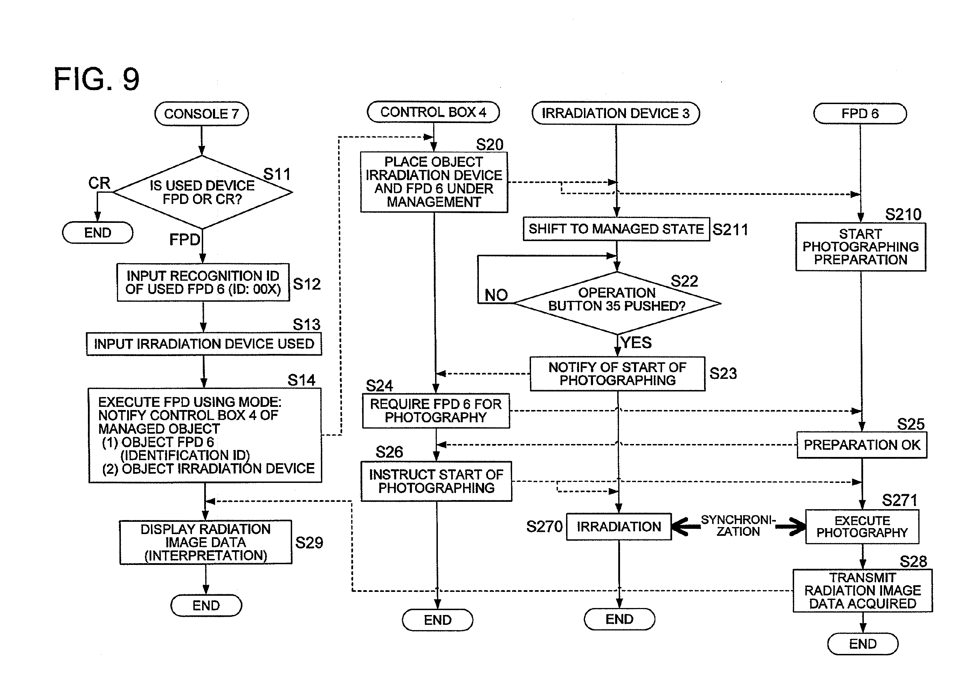

Fig. 9 is a diagram for explaining the control flow executed by the radiography system. Because of this control flow, it is possible to achieve synchronization between the FPD 6 and the radiation emitting device. This is explained below.

In Step S11, a decision is made as to whether the device to be used for radiography is an FPD that requires synchronization with the radiation emitting device 3 or a CR cassette that does not require synchronization. The instruction by the operator of using an FPD corresponds to the "radiography instruction by the operator of the fact that a radiation detecting device (FPD) is to be used".

If it is an FPD (FPD in Step S11), the FPD 6 and the radiation emitting device 3 to be used in Step S12 and Step S13 are identified. This identification is done by the operator making an input instruction to the console 7.

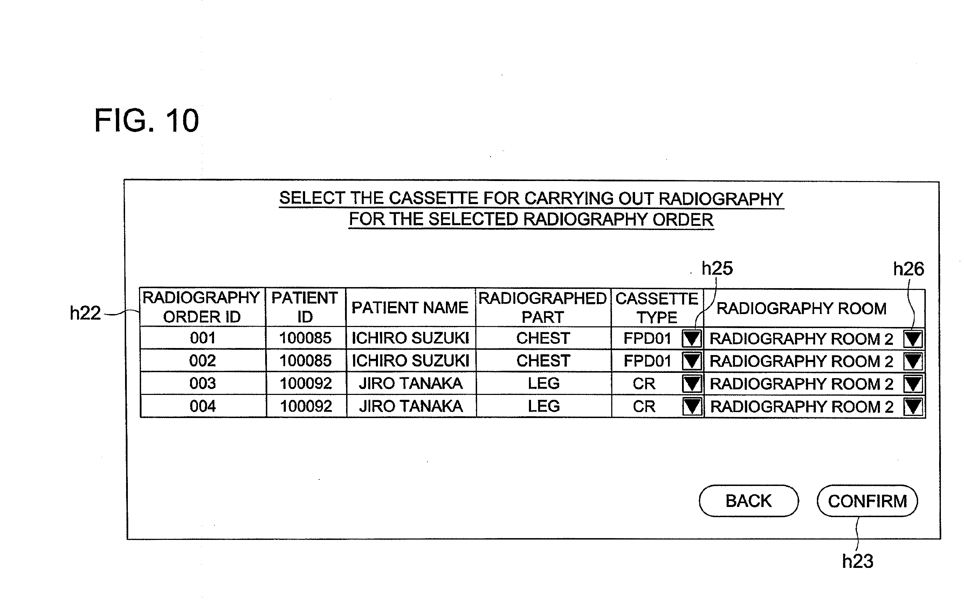

An example of the procedure of identifying is explained based on Fig. 10. Fig. 10 is an example of the settings screen displayed in the display section 77. Through this settings screen are carried out the radiography instruction of executing radiography of the order information that has the information of the patient, the radiographed part of the body, the radiographing direction, etc., using an FPD (radiation detecting device) among either a CR cassette an FPD, and the identification of the radiation emitting device 3 used for radiography. In addition, when an FPD 6 is to be used as the radiation image detecting device, even the selection of its identification code ID is also made at the same time.

The radiography order is being displayed in the display column h22. The selection of the type and identification code ID of device (type of cassette) corresponding to this radiography order can be made by operating the selection button h25. Further, by operating the selection button h25, it is possible to select the radiography room. In the example shown in this figure, regarding the radiography related to the radiography order ID No. "001" and "002", a selection is being made of using the FPD 6 with the identification code ID "01", and radiographing in the radiography room 2. Next, these selections are confirmed when the confirmation button h23 is pressed.

In the example of the settings screen of the display section 77 shown in Fig.10, due to the operation of the selection button h25 and the confirmation button h23, inputs are made of the "radiography instruction by the operator of the fact that a radiation image detecting device (FPD) is to be used" and of the "selection instruction of the radiation image detecting device (identification code ID) used for radiography".

Further, the "selection instruction of the radiation emitting device used for radiography" is issued by making the operations of pressing the selection button h25 and the confirmation button h23. In the example shown in Fig. 2, since there is only one radiation emitting device 3 in the radiography room 2 (radiography room 100b), by identifying the radiography room, the fact of using the radiation emitting device 3 present in that room is being identified. As another example, when there is a plurality of radiation emitting devices 3 in a single room, it is possible to display the identification numbers, etc. of the radiation emitting devices and to identify the radiation emitting device.

From the settings screen shown in Fig. 10, when the input of the identification code ID of the FPD 6 (Step S12) and the input of the radiation emitting device 3 to be used is made (Step S13), the console 7 determines that the mode of using an FPD is to be executed, and in Step S14, notifies the control box 4 of the targets of management obtained in Step S12 and Step S13.

Further, in an embodiment in which a plurality of control boxes 4 is provided (for example, Fig. 4), the corresponding control box 4 is identified from the target radiation emitting device 3, and the notification of Step 14 is made to that control box 4.

In addition, because the FPD 6 to be used is identified in Step S12, it is also possible to carry out "position detection" of that FPD 6 as has been described earlier. Further, it is also good to carry out (1) control so as to make the radiation emitting device 3 corresponding to the detected radiography room as the radiation emitting device 3 to be used, or to carry out (2) control so that a warning message is displayed when the FPD 6 is present in a radiography room different from the radiography room in which the radiation emitting device 3 to be used is present. Because of the former (1), it is possible to omit the operation of Step S13, and because of the latter (2), it is possible to prevent radiography mistakes due to using a wrong FPD 6.

In Step S20, the control box 4, based on the notification in Step S14, issues notifications to the target radiation emitting device 3 (installed in the radiography room 2) and to the FPD 6 with the target identification code ID that they are under management.

The FPD 6 that has received the notification goes into the "charge reset state" and starts the preparations for radiography (Step S210).

In the radiation emitting device 3 that has received the notification, a transition is made to the managed state (Step S211). In the managed state, even when the operation button 35 is pressed by the operator in Step S22, radiation emission is not started immediately, but a radiation start notification is issued to the control box 4 that is managing that device (Step S23). Further, at this time, it is also possible to put it in the preparatory state for carrying out rotation of the rotating anode 33 (see Fig. 1).

In Step S24, based on the notification in Step S23, the control box 4 sends a radiography request to the FPD 6. If the preparations have been made in the FPD 6, a notification of that fact is issued (Step S25).

In Step S26, the control box 4 gives notification of the start radiography instruction to the radiation emitting device 3 and the FPD 6. Based on that notification, the radiation emitting device 3 starts the emission of radiation (Step S270), and in the FPD 6, after a prescribed period of time has elapsed in the "accumulation enabled state", the state is changed to the "charge reading state", and the acquisition of the radiation image is carried out. Further, even regarding the stopping of the emission of radiation, a notification is given when it is determined on the FPD 6 side that a prescribed amount of exposure has been obtained, and the control box 4 receiving that notification can send a stop signal to the radiation emitting device 3.

In Step S28, the FPD 6 transmits the obtained radiation image data to the console 7 that notified the control box 4 of the target of management in Step S14. In the console 7, depending on the necessity, computation processing such as gain correction, offset correction, gradation processing, frequency emphasis processing, or granulation suppression processing, etc., are carried out. Next, the radiation image data after computation processing is made to be displayed in the display section 77 (Step S29).

Further, although the control flow shown in Fig. 9 was explained regarding the control flow in the case of a radiography system mainly provided with a plurality of FPDs 6, and a plurality of radiation emitting devices 3, such as in the configuration examples shown in Fig. 2 to Fig. 4, it can also be applied to a radiography system configured from a single FPD 6 or a single radiation emitting device 3 such as the configuration shown in Fig. 1. In this case, it is possible to omit the control of selecting radiography from among a plurality of FPDs 6 and a plurality of radiation emitting devices 3.

According to the present embodiment, even when a different imaging device (portable FPD) is added to an existing system (CR system), by having a configuration in which a control box corresponding to the imaging device is added, it is possible, without adding changes to the part of coordinating with the higher level such as an RIS or an HIS, etc. (establishing correspondence between the radiography order information and the radiographed image, etc.), within an existing system, to limit the range of its effect, and to reduce the scope of system debugging at the time of functional expansion of the system, and to carry them out easily.

In the fifth and sixth embodiments described below, unlike the embodiments descried above, an interface with the radiation emitting device is not necessary for generating the image data, and the systems are those that use self controlling type FPDs in which the FPD itself carries out control of the start of emitting radiation, accumulation of image data, and starting of reading the image data.

### [FPD Provided with a Current Detection Means]

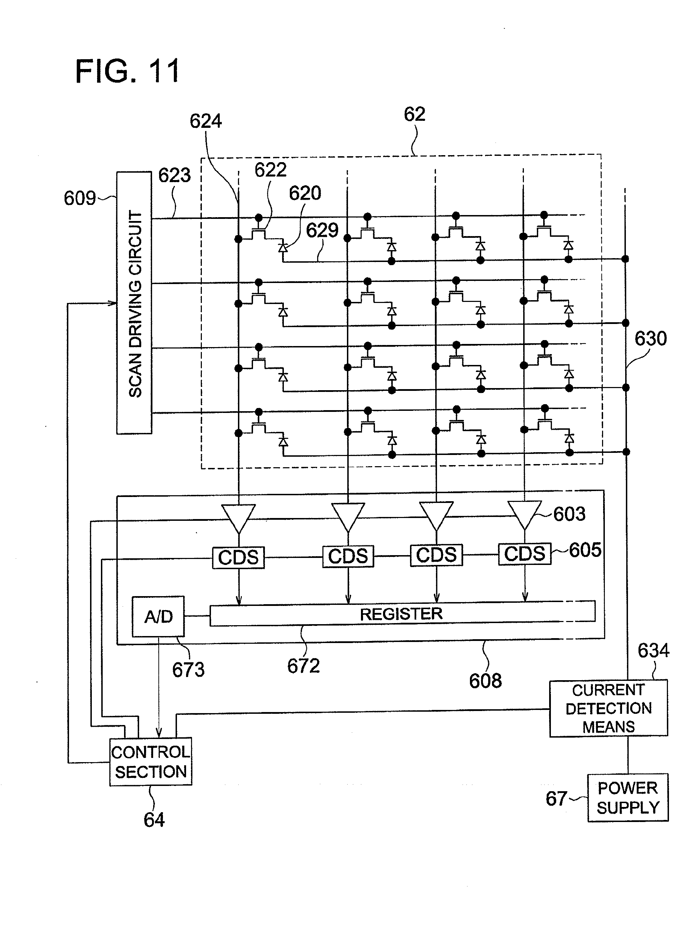

Next, based on Fig. 11 to Fig. 14, the radiography systems according to a fifth and a sixth embodiment are described.

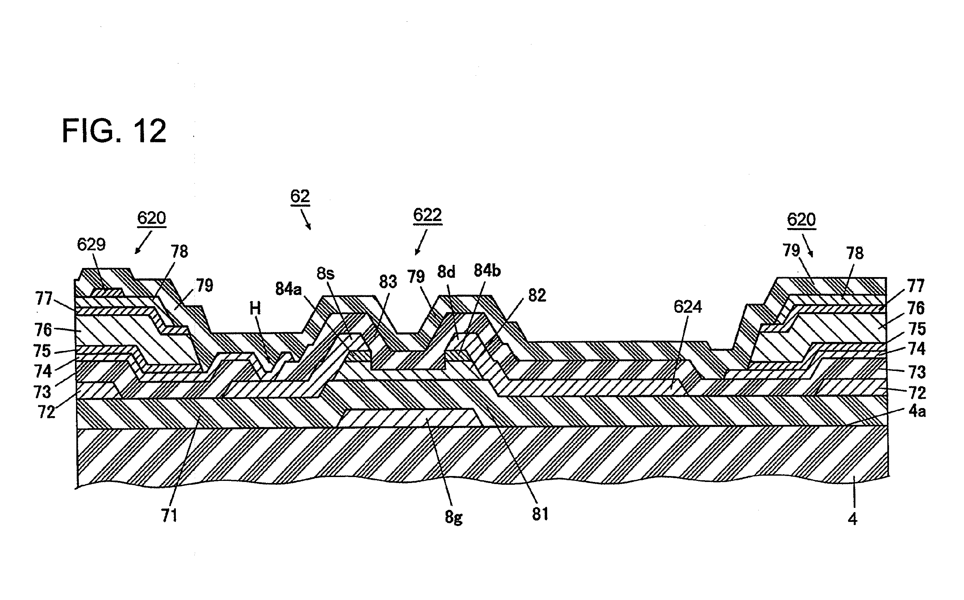

Fig. 11 is a schematic diagram showing the imaging panel 62 and its peripheral circuits in an FPD 6 in the fifth and sixth embodiments. The explanations of the configuration parts common to Fig. 8 described above are substituted by assigning identical signs. Fig. 12 is a cross-sectional view diagram of the imaging panel 62 shown in Fig. 11.

In an FPD 6 in the embodiment shown in Fig. 11 and Fig. 12, a current detection means 634 has been provided, and the starting of radiation emission and the stopping of radiation emission by the radiation emitting device 3 are detected by that current detection means 634.

The imaging panel 62 shown in Fig. 11 and Fig. 12 is formed on the surface 4a on the light receiving side of a substrate 4, by forming superimposed layers of the gate electrodes 8g of TFT 622 (constituted by signs 81, 82, 83, 84a, 84b, 8d, and 8g) prepared from Al, Cr, etc. and of the scanning lines 623 in an integrated manner, in the top part of the gate electrode 8g on the gate insulation layer 81 made of silicon nitride (SiNₓ), etc. and superimposed on the gate electrode 8g and the surface 4a are formed the source electrodes 8s connected to the first electrode 74 of the light receiving element 620 (constituted by signs 72 to 79) via a semiconductor layer 82 made of hydrogenated amorphous silicon (a-Si), and forming the signal lines 624 integrally with the drain electrodes 8d in a superimposing manner.

The source electrodes 8s and the drain electrodes 8d are separated by a first passivation layer 83 made of silicon nitride (SiNₓ), etc., and in addition, both electrodes 8s and 8d are covered from above by the first passivation layer 83. In addition, between the semiconductor layer 82 and the source electrode 8s or the drain electrode 8d are superimposed respectively the ohmic contacts 84a and 84b formed from hydrogenated amorphous silicon made into n-type by doping with a group VI element. Thus a TFT 622 such as the above is formed.

Further, in the part of the light receiving element 620, a layer of Al or Cr, etc. is formed on said insulating layer 71 formed integrally with said gate insulating layer 81 on the surface 4a of the substrate 4 thereby forming an auxiliary electrode 72, and a layer of the first electrodes 74 made of Al or Cr, Mo, etc. is formed on the auxiliary electrodes 72 with an intervening insulating layer 73 that is formed integrally with said first passivation layer 83. The first electrodes 74 are connected to the source electrodes 8s of the TFT 622 via the hole H formed in the first passivation layer 83.

On the first electrodes 74 an n-layer 75 of hydrogenated amorphous silicon made to become n-type by doping with a Group VI element, an i-layer 76 which is the conversion layer formed from hydrogenated amorphous silicon, and a p-layer 77 of hydrogenated amorphous silicon made to become p-type by doping with a Group III element are formed in the sequence from below to above,

When the electromagnetic waves converted in the light emitting layer 63 that has received the emitted radiation are incident from above, electron-hole pairs are generated in the i-layer 76. The light receiving element 620 converts the electromagnetic waves from the light emitting layer 63 into electric charge in this manner. Further, the sequence of the p-layer 75, the i-layer 76, and the n-layer 77 can also be reverse of the above,

Above the p-layer 77, a layer of the second electrodes 78 made as transparent electrodes such as ITO, etc. is formed so that the incident electromagnetic waves reach the i-layer 76, etc. The light receiving element 620 is formed in the above manner.

Further, on the top surface of the second electrode 78 of the light receiving element 620 a bias line 629 for applying a reverse bias voltage to the light receiving elements 620 via the second electrode 78 is connected. Further, the second electrode 78 or the bias line 629 of the light receiving element 620, the first electrode 74 extended towards the TFT 622, the first passivation layer 83 of the TFT 622, etc., that is, the top surface parts of the light receiving element 620 and the TFT 622 are covered from above by a second passivation layer 79 formed from silicon nitride (SiNₓ), etc.

In the present embodiment, as is shown in Fig. 11, one bias line 629 is connected to a plurality of light receiving elements 620 respectively arranged in the shape of a column, and each bias line 629 is placed so that it intersects the respective signal lines 624 at right angles. In addition, all the bias lines 629 are joined to one connection line 630 at a position on the outside of the detection part P of the substrate 4. The bias lines 629 and the connection line 630 are formed from a metallic wire having small electrical resistance.

The one end of the electrodes of the different light receiving elements 620 of the imaging panel 62 is connected respectively to a bias line 629, and all the bias lines 629 are joined to one connection line 630. The connection line 630 is connected to the power supply 67 via a current detection means 634. The power supply 67 applies negative bias voltage to each of the light receiving elements 620 via each of the bias lines 629.

To the output terminal of the amplifier circuit 603 is connected a correlated double sampling circuit (hereinafter referred to as CDS) 605. Here, the correlated double sampling circuit 605 is a circuit that removes (reduces) the noise at the time of resetting the capacitor by taking the difference between a first voltage value of the capacitor of the charge to voltage conversion circuit after resetting and a second voltage value read out after radiography (after emitting the radiation). Next, the procedure of detecting the starting and stopping of the emission of radiation by the control section 64 and the current detection means 634 is explained below.

In the embodiment shown in Fig. 11, when a negative voltage that is the reverse bias voltage is applied to the second electrode 78 of the light receiving element 620 via the bias line 629, a potential gradient is generated inside the light receiving elements 620. In this condition, when radiation is emitted from a radiation source such as a radiation emitting device 3, etc., and the electromagnetic waves converted from the radiation by the light receiving layer 63 that has received the radiation are incident on the i-layer 76 (the conversion layer) of the light receiving elements 620, electron-hole pairs are generated inside the i-layer 76.

Next, among the generated electro-hole pairs, while the electrons move following the potential gradient towards the first electrode 74 which is at a high potential, since the gate of the TFT 622 is closed, the electrons get accumulated in the first electrode 74 or near the first electrode 74 inside the i-layer 76. Since electron-hole pairs are generated in the i-layer 76 of the light receiving element 620 in proportion to the number of photons of the electromagnetic waves, inside the light receiving element 620, an amount of electrons are accumulated that is in proportion to the amount of electromagnetic waves that are incident.

On the other hand, among the generated electron-hole pairs, the holes move following the potential gradient towards the second electrode 78 which is at a low potential, and flow to the bias line 629 through the second electrode 78. As is shown in Fig. 11, the holes that flow out from this light receiving element 620 and flow through the bias line 629 are detected as current by the current detection means 634.

Since even the holes flowing through the bias line 629 are generated as the number of electron-hole pairs generated in proportion to the number of photons of the electromagnetic radiation incident in the i-layer 76 of the light receiving element 620, the same quantity of holes flow in the bias line 629 as the quantity of electrons accumulated inside the light receiving element 620 according to the amount of incident electromagnetic waves. In other words, the currents flowing through the bias lines 629 are collected together in the connection line 630, and flow through the connection line 630 towards the current detection means 634.

When based on the principle of generation of electric charge in the above light receiving element 620, in the stage prior to that of emission of radiation during which radiation or electromagnetic waves are not incident on the i-layer 76 of the light receiving element 620, ideally although no current flows through the bias line 629 or the connection line 630, in actuality, dark current is generated in the light receiving element 620, and a very small current is detected by the current detection means 634.

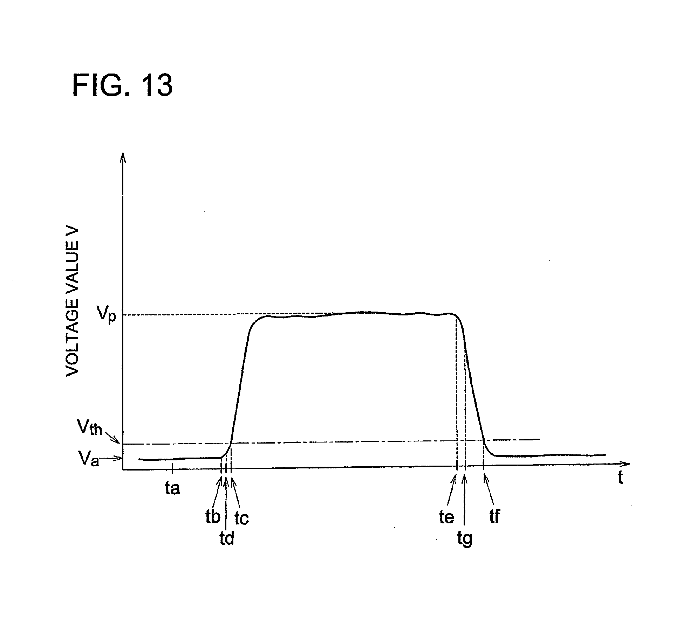

Fig. 13 shows and example of the change with time of the voltage value output by converting the current into voltage in the current detection means 634. As has been explained before, in the fifth and sixth embodiments, since the current detection means 634 converts the current flowing through the connection line 630 into a voltage value and outputs it, even in the stage prior to that of emission of radiation during which radiation or electromagnetic waves are not incident on the i-layer 76 of the light receiving element 620, as shown by the instant of time ta in Fig. 13, a very small but not zero voltage value Va is input from the current detection means 634 to the control section 64.

Next, the emission of radiation from the radiation source is started, electron-hole pairs are generated inside each of the light receiving elements 620, holes are carried to the current detection means 634 by passing through the bias lines 629 and the connection line 630. Because of this, as is shown at the instant of time tb in Fig. 13, the voltage value V output from the current detection means 634 increases. Therefore, in the present embodiment, the control section 64 detects the start of emission of radiation by detecting the voltage value V output from the current detection means 634 starting to increase in large quantities.

Regarding the start of emission of radiation based on the increase in the voltage value V, it is also possible to configure so as to detect that the emission of radiation has started at the instant of time tc at which the voltage value V exceeds a prescribed threshold value Vth, or else, it is also possible to configure so as to detect that the emission of radiation has started at the instant of time td when the time differential value of the voltage value V exceeds a prescribed value.

Further, when the emission of radiation from the radiation source ends, at that time, the generation of electron-hole pairs inside each of the light receiving elements 620, and holes will not be supplied to the bias lines 629. Because of this, as is shown at the instant of time te in Fig. 13, the voltage value V output from the current detection means 634 starts to decrease. Therefore, in the present embodiment, the control section 64 detects the end of the emission of radiation by detecting that the voltage value V output from the current detection means 634 has decreased.

Regarding the end of emission of radiation due to the decrease in the voltage value V, it is also possible to configure so that the emission of radiation is detected to have stopped at the instant of time tf when the voltage value V has fallen below the prescribed threshold value Vth described above, or else, it is also possible to configure so that the emission of radiation is detected to have stopped at the instant of time tg when the time differential value of the voltage value V has crossed a negative threshold value towards the negative side.

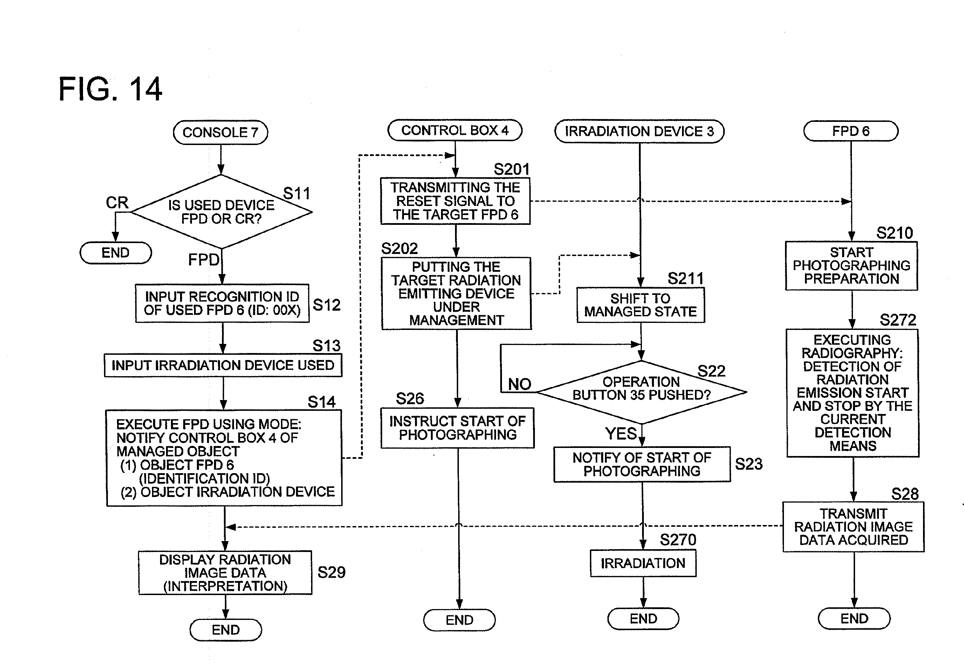

Next, based on Fig. 14, the control flow executed by the radiography system in the fifth embodiment is explained below. In this figure, the explanations regarding the controls common to the control flow shown in Fig. 9 are substituted by assigning the same signs. In the fifth embodiment and the succeeding sixth embodiment, the FPD 6 has been configured so that it by itself detects the starting and stopping of the emission of radiation. From this, there is no necessity for the control box 4 to achieve synchronization with the radiation emitting device 3 and FPD 6 at the time of imaging.

In Step S201 in Fig. 14, based on the notification in Step S14, the control box 4 transmits a reset signal as a reset instruction to the target FPD 6. The FPD that has received that reset signal goes into the "charge reset state" and starts the preparations for imaging (Step S210). In the present embodiment, although imaging is possible even if the transmission of the reset signal is not made, in order to remove noise due to dark current, it is desirable to carry out resetting of the charge that has accumulated up to that time by transmitting a reset signal.

Further, in Step S202, the control box 4 puts the target radiation emitting device 3 of the target radiation under management. In the radiation emitting device 3 that has received the notification, a transition is made to the managed state (Step S211).

The Steps S22, S23, and S26 of the radiation emitting device 3 are the same as in the control flow shown in Fig. 9 and their explanations are omitted. Based on the notification of the imaging start instruction of Step S26, the radiation emitting device 3 carries out emission of radiation for a prescribed duration at a prescribed radiation intensity (Step S270). Further, these prescribed duration and prescribed radiation intensity can be carried out as described above from the operation section 34, or the configuration can be made so that they are set automatically by the control box 4 according to the sensitivity of the FPD 6 used using a conversion table according to the body part information included in the radiography order information.

In Step S272, the FPD 6, the control section 64 detects the starting and stopping of the emission of radiation based on the increase or decrease in the current flowing through the bias line 629 detected by the current detection means 634. Next, the control section 64 puts the FPD 6 in the "accumulation enabled state" upon detecting the start of emission of radiation, and puts it in the "charge reading state" upon detecting the stopping of the emission of radiation. In Step S28, since the further controls are the same as in Fig. 9, their explanations are omitted here.

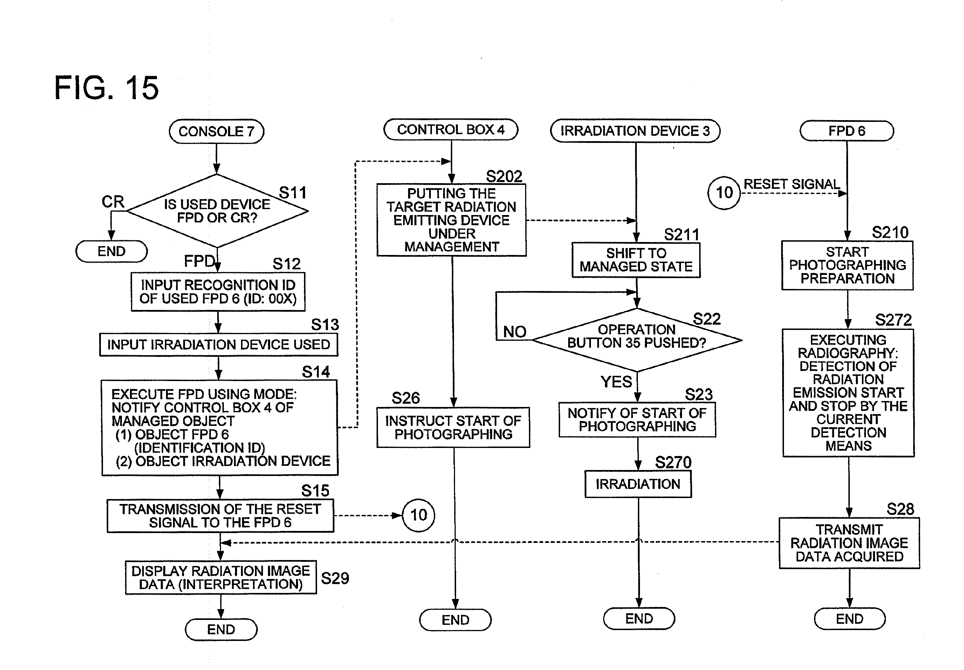

Next, the control flow executed by the radiography system in the sixth embodiment is explained based on Fig. 15. Even in the sixth embodiment, the FPD 6 has been configured so that it by itself detects the starting and stopping of the emission of radiation. The difference with the fifth embodiment is that the transmission of the reset signal to the FPD 6 is carried out by the console 7 functioning as a control terminal.

As is shown in Fig. 15, in Step S15, the console 7 transmits the reset signal to the target FPD 6. In the FPD 6, in the FPD that has received that reset signal, a transition is made to the "charge reset state" and the preparations for imaging are started (Step S210). The control thereafter is the same as the control flow shown in Fig. 14 and its explanation is omitted.

According to the fifth and sixth embodiments, even in the case in which a different imaging device (portable FPD) is added to an existing system (CR system), by making the configuration one in which a control box corresponding to the imaging device is added, it is possible to limit the range of its effect without making changes in the parts coordinating with a higher level side such as an RIS or an HIS, etc. (establishing correspondence between the radiography order information and the radiographed image), it is possible to reduce the scope of system debugging during a functional expansion of the system, and it is possible to carry them out easily. In addition, it becomes possible to use an FPD that is configured so that it by itself detects the starting and stopping of the emission of radiation, it becomes possible to make the radiation emitting timing the same as that when a radiography technician carries out radiography during the radiography using an FPD and during the radiography using a CR, and it is desirable because it is possible to maintain the conventional patient radiographing technique as it is.

Aspects of the invention are set out in the following series of numbered clauses.
1. A radiographic imaging system comprising:
   a portable type radiation image detecting device that carries out radiography based on a radiation from a radiation emitting device provided in a radiography room and obtains radiation image data;
   a control terminal which is provided with an operation section and a display section, acquires radiography order information, and establishes correspondence between the acquired radiography order information and radiation image data generated by said radiation image detecting device; and
   a managing means which controls at least one radiation emitting device of the radiation emitting device,
   wherein each of said radiation image detecting device, said control terminal and said managing means is possible to mutually communicate via communication lines,
   wherein said control terminal sends notification of a radiography instruction to said managing means, based on an input of a radiography instruction by an operator to the operating section, the radiography instruction is an instruction to the effect that said radiation image detecting device is to be used; and
   wherein said managing means carries out radiography by controlling the radiation emitting device or controlling the radiation emitting device and said radiation image detecting device based on the notification.
2. The radiographic imaging system according to clause 1, wherein said managing means controls a timing of emission of radiation of said radiation emitting device and a timing of obtaining radiation image data of said radiation image detecting device.
3. The radiographic imaging system according to clause 1 or 2,
   wherein said managing means is capable of controlling a plurality of the radiation emitting devices,
   wherein said control terminal sends notification of the radiography instruction and a selection instruction of the radiation emitting device to be used for radiography to said managing means, based on an input of the radiography instruction and an input of the selection instruction of the radiation emitting device to be used by an operator to the operating section; and
   wherein said managing means carries out radiography by controlling the selected radiation emitting device and said radiation image detecting device based on the notification.
4. The radiographic imaging system according to any one of clause 1 to 3,
   wherein the radiographic imaging system comprises a plurality of radiation image detecting devices to which identification IDs are assigned,
   wherein said control terminal sends notification of the radiography instruction and a selection instruction of a radiation image detecting device to be used for radiography to said managing means, based on an input of the radiography instruction and an input of the selection instruction of the radiation image detecting device to be used by an operator to the operating section; and
   wherein said managing means carries out radiography by controlling the radiation emitting device and the selected radiation image detecting device based on the notification.
5. The radiographic imaging system according to any one of clauses 1 to 4,
   wherein, said radiation image detecting device is provided with a wireless communication section that can carry out wireless communication;
   wherein said managing means, executes radiography by controlling said radiation image detecting device through wireless communication; and
   wherein said radiation image detecting device carries out transmission of the generated radiation image data to said control terminal by wireless communication.
6. The radiographic imaging system according to any one of clauses 1 to 5, wherein the radiographic imaging system comprises a plurality of said control terminals and at least one of the plurality of control terminals and said managing means are connected by a wireless communication circuit.
7. The radiation imaging system according to clause 1,
   wherein said radiation image detecting device comprises:
   a plurality of detection elements that convert an energy corresponding to an amount of radiation impinging on them into an amount of electrical charge;
   a bias line that applies a reverse bias voltage to each of said detection elements;
   a power supply that applies a reverse bias voltage to said detection elements via said bias line;
   a current detection means that detects a current flowing through said bias line; and
   a control means that detects a start and an end of emission of said radiation based on an increase or a decrease in the current flowing through said bias line detected by said current detection means,
   wherein said managing means, based on said notification, carries out notification of a reset instruction to said radiation image detecting device.
8. The radiation imaging system according to clause 1,
   wherein said radiation image detecting device comprises:
   a plurality of detection elements that convert an energy corresponding to an amount of radiation impinging on them into an amount of electrical charge;
   a bias line that applies a reverse bias voltage to each of said detection elements;
   a power supply that applies a reverse bias voltage to said detection elements via said bias line;
   a current detection means that detects a current flowing through said bias line; and
   a control means that detects a start and an end of emission of said radiation based on an increase or a decrease in the current flowing through said bias line detected by said current detection means,
   wherein said control terminal, based on said notification, carries out notification of a reset instruction to said radiation image detecting device.

## Claims

1. A radiography system comprising:
one or more radiation emitting devices;
one or more portable type radiation image detecting devices for generating radiation image data by detecting radiation emitted from the one or more radiation emitting devices; and
a console configured to obtain radiation image data from the one or more portable type radiation image detecting devices;
wherein each the one or more portable type radiation image detecting devices is configured:
to detect the starting of the emission of X-ray radiation by a radiation emitting device of the system, whereby there is no necessity to achieve synchronization with the radiation emitting device and portable type radiation image detecting device at the time of imaging; and
to transmit to the console radiation image data accumulated on detection of the starting of the emission of X-ray radiation.

2. The system of claim 1 comprising a plurality of said portable type radiation image detecting devices.

3. The system of claim 1 or claim 2 comprising a plurality of said radiation emitting devices.

4. The system of any preceding claim, wherein at least some of the portable type radiation image detecting devices are portable type flat panel detectors.

5. The system of claim 4, wherein each of the portable type radiation image detecting devices is a portable type flat panel detector.

6. The system of any preceding claim, the portable type radiation image detecting devices comprise:
a plurality of detection elements that convert an energy corresponding to an amount of radiation impinging on them into an amount of electrical charge;
a bias line that applies a reverse bias voltage to each of said detection elements;
a power supply that applies a reverse bias voltage to said detection elements via said bias line;
a current detection means that detects a current flowing through said bias line; and
a control means that detects a start and an end of emission of said radiation based on an increase or a decrease in the current flowing through said bias line detected by said current detection means.

7. The system of any preceding claim, further comprising a control box connected to each portable type radiation image detecting device, each radiation emitting device and the console.
